# EUROPEAN PATENT SPECIFICATION

(11) **EP 3 152 325 B1**
(45) Date of publication and mention of the grant of the patent: **04.09.2019**
(21) Application number: 15728100.7
(22) Date of filing: 03.06.2015
(51) Int. Cl.: C12Q 1/68

(54) **METHOD OF DIAGNOSING TRIGEMINAL NEURALGIA**
VERFAHREN ZUR DIAGNOSE VON TRIGEMINAL NEURALGIA
MÉTHODE DE DIAGNOSTIC DE LA NEURALGIE TRIGÉMINALE

(30) Priority: 03.06.2014 GB 201409851
(43) Date of publication of application: 12.04.2017
(73) Proprietor: Convergence Pharmaceuticals Limited, London WC1V 6XX (GB)
(72) Inventor: MORISSET, Valerie, Cambridge Cambridgeshire CB22 3AT (GB)
(74) Representative: Miller, David James
(86) International application number: PCT/GB2015/051618
(87) International publication number: WO 2015/185924

(56) References cited:
- US-A1- 2011 183 335
- US-A1- 2013 296 182
- OPHOFF R A ET AL: "FAMILIAL HEMIPLEGIC MIGRAINE AND EPISODIC ATAXIA TYPE-2 ARE CAUSED BY MUTATIONS IN THE CA2+ CHANNEL GENE CACNL1A4", CELL, CELL PRESS, US, vol. 87, no. 3, 1 November 1996 (1996-11-01), pages 543-552, XP000647699, ISSN: 0092-8674, DOI: 10.1016/S0092-8674(00)81373-2
- D'ONOFRIO M ET AL: "The interplay of two single nucleotide polymorphisms in the CACNA1A gene may contribute to migraine susceptibility", NEUROSCIENCE LETTERS, LIMERICK, IE, vol. 453, no. 1, 27 March 2009 (2009-03-27), pages 12-15, XP026000721, ISSN: 0304-3940, DOI: 10.1016/J.NEULET.2009.01.081 [retrieved on 2009-02-04]
- BOUKJE DE VRIES ET AL: "Molecular genetics of migraine", HUMAN GENETICS, SPRINGER, BERLIN, DE, vol. 126, no. 1, 20 May 2009 (2009-05-20), pages 115-132, XP019740876, ISSN: 1432-1203, DOI: 10.1007/S00439-009-0684-Z
- HULLUGUNDI S K ET AL: "A hyperexcitability phenotype in mouse trigeminal sensory neurons expressing the R192QCacna1amissense mutation of familial hemiplegic migraine type-1", NEUROSCIENCE, vol. 266, 25 April 2014 (2014-04-25), pages 244-254, XP028847789, ISSN: 0306-4522, DOI: 10.1016/J.NEUROSCIENCE.2014.02.020
- VAN DEN MAAGDENBERG ARN M J M ET AL: "Migraine: gene mutations and functional consequences", CURRENT OPINION IN NEUROLOGY, RAPID SCIENCE PUBLISHERS, LONDON, GB, vol. 20, no. 3, 1 June 2007 (2007-06-01), pages 299-305, XP009185710, ISSN: 1350-7540
- GOADSBY ET AL: "Recent advances in understanding migraine mechanisms, molecules and therapeutics", TRENDS IN MOLECULAR MEDICINE, ELSEVIER CURRENT TRENDS, GB, vol. 13, no. 1, 4 January 2007 (2007-01-04), pages 39-44, XP005822073, ISSN: 1471-4914, DOI: 10.1016/J.MOLMED.2006.11.005
- RAMIN RAOUF ET AL: "Pain as a channelopathy", JOURNAL OF CLINICAL INVESTIGATION, vol. 120, no. 11, 1 November 2010 (2010-11-01), pages 3745-3752, XP055205657, ISSN: 0021-9738, DOI: 10.1172/JCI43158
- SULAYMAN D. DIB-HAJJ ET AL: "Sodium Channels in Normal and Pathological Pain", ANNUAL REVIEW OF NEUROSCIENCE, vol. 33, no. 1, 1 June 2010 (2010-06-01), pages 325-347, XP055205643, ISSN: 0147-006X, DOI: 10.1146/annurev-neuro-060909-153234 cited in the application
- CAROLINE R. FERTLEMAN ET AL: "SCN9A Mutations in Paroxysmal Extreme Pain Disorder: Allelic Variants Underlie Distinct Channel Defects and Phenotypes", NEURON, vol. 52, no. 5, 1 December 2006 (2006-12-01), pages 767-774, XP055206248, ISSN: 0896-6273, DOI: 10.1016/j.neuron.2006.10.006
- JOANNA M ZAKRZEWSKA ET AL: "Novel design for a phase IIa placebo-controlled, double-blind randomized withdrawal study to evaluate the safety and efficacy of CNV1014802 in patients with trigeminal neuralgia", TRIALS, BIOMED CENTRAL, LONDON, GB, vol. 14, no. 1, 23 November 2013 (2013-11-23), page 402, XP021170165, ISSN: 1745-6215, DOI: 10.1186/1745-6215-14-402
- NACHBAUER WOLFGANG ET AL: "Episodic ataxia type 2: phenotype characteristics of a novel CACNA1A mutation and review of the literature", JOURNAL OF NEUROLOGY - ZEITSCHRIFT FUER NEUROLOGIE, SPRINGER VERLAG, BERLIN, DE, vol. 261, no. 5, 22 March 2014 (2014-03-22), pages 983-991, XP035307078, ISSN: 0340-5354, DOI: 10.1007/S00415-014-7310-2 [retrieved on 2014-03-22]

## Description

### FIELD OF THE INVENTION

The invention relates to a method of diagnosing trigeminal neuralgia in a human subject and also provides a method of identifying patients most likely to respond to therapy and to kits for use in said methods.

### BACKGROUND OF THE INVENTION

Paroxysmal pain disorders refer to pain disorders which are characterised by attacks of pain. Such disorders typically include trigeminal neuralgia (TN), inherited erythromelalgia (IEM), paroxysmal extreme pain disorder (PEPD) and small fiber neuropathy (SFN).

Trigeminal neuralgia (TN) is an uncommon episodic severe facial pain condition with an incidence of 4.0 to 4.7 per 100,000 persons per year (Obermann et al (2007) Cephalalgia 27(6):504-509; Katusic et al (1991) Neuroepidemiology 10(5-6):276-281). Trigeminal neuralgia can appear at any age, but disease onset is over 40 years of age in over 90% of cases with peak onset between 50 and 70 years of age (Katusic *et al* supra; van Kleef M et al (2009) Trigeminal neuralgia. Pain Pract 9(4):252-259). However, recent data from primary care practice data validated by experts suggest an incidence rate of 12.6 per 100,000 person years with a mean age at diagnosis of 51.5 (SD 17.6) with a female predominance 71% (Koopman et al (2009) Pain 147(1-3):122-127). The classification system of the International Headache Association aims at establishing TN diagnostic criteria based on etiology (IHS: IHS Classification ICHD-111 Beta version. 2013, 33(9):629-808). Yet, there is a problem in distinguishing primary and secondary TN, because unless a patient with normal neuroimaging comes to an operation, it remains unclear if his/her TN is caused by a vascular compression. To adjust for this diagnostic uncertainty, the term "classical" neuralgia is currently used for cases with normal neuroimaging and potential compression of the proximal trigeminal nerve root by a vascular loop. Classical TN is the most common TN type, and it is thought that secondary demyelination, probably mediated by microvascular ischemic damage, results in a lowered excitability threshold of affected neurons. This promotes inappropriate ectopic generation of spontaneous nerve impulses together with abnormal nonsynaptic ephatic transmission to adjacent neurons (Obermann et al (2007) Neurology 69(9):835-841; Prasad and Galetta (2009) Neurologist 15(2):87-94).

Unlike many other neuropathic pains, TN results in recurrent paroxysms of short-lasting but very severe pain in the distribution of one or more branches of the trigeminal nerve. In patients with the classical type, the attacks come on suddenly, last up to 2 min and disappear suddenly. Between attacks, patients are usually asymptomatic, although there may be a slight after pain (Zakrzewska JM (2002) Clin J Pain 18(1):14-21). Numerous attacks occur a day, but currently there are no studies detailing how many paroxysms occur a day. In a recent RCT of botulinum toxin A in 42 TN patients, the frequency of paroxysms was measured, and the average was 20 a day, but the range was 4-100 (Wu et al (2012) Cephalalgia 32(6):443-450). Patients also report that these can be so frequent that it seems like one long attack. The attacks can vary in severity and be described as shooting, electric shocks scoring maximum scores on scales or just "twinges." These attacks can then go on for weeks or months. Especially in the early stages of the disorder, it is very common for the attacks to stop completely and for patients to have weeks, months or even years of no pain (Rothman and Monson (1973) J Chronic Dis 26(5):303-309). However, over time the remission periods get shorter. Currently, there are no natural history studies or known prognostic factors to help determine how long these periods can be, although Taylor et al. (Postgrad Med J 1981, 57:16-18) showed that carbamazepine became less effective with time. This may be due to the natural course of the disease, but may also be vested in the autoinduction pharmacological properties of carbamazepine itself. Pain is provoked by light touch activities, e.g., washing, eating, talking and cold winds, but spontaneous attacks of pain also occur. One study that measured the occurrence of evoked or spontaneous pain suggested that oxcarbazepine was more effective at reducing the former (Beydoun *et al* (2002), 58:p02.083). Although attacks of pain occur at night, these are less common. Thus, even when pain free, patients live in fear of pain return. These features therefore make it difficult to evaluate the effect of treatments. The diagnostic criteria for TN most frequently used are those of the International Headache Society, the International Classification of Headache Disorders (ICHD) (Anon (2004) Cephalalgia 24(Suppl 1):9-160), and these have now been updated with clearer specification of the variants of TN, i.e., those patients who may have in addition to the shooting stabbing pain a more prolonged pain, and these are thought to have a potentially different pathophysiology (Anon (2013) Cephalalgia 33(9):629-808).

Current guidelines in trigeminal neuralgia management recommend sodium channel blockers, such as carbamazepine or oxcarbazepine, as the first-line treatment. However, the currently available drugs are often associated with poor tolerability resulting in sub-optimal pain control.

Inherited erythromelalgia (IEM) is characterized clinically by burning pain and redness that is usually focused on the distal extremities, precipitated by mild warmth and relieved by cooling, and is caused by Nav1.7 mutations that hyperpolarize activation, slow deactivation, and enhance the channel ramp response (Dib-Hajj et al (2010) Annu Rev Neurosci 33:325-347).

Paroxysmal extreme pain disorder (PEPD) is characterized by perirectal, periocular or perimandibular pain, often triggered by defecation or lower body stimulation (Fertleman et al (2007) Neurology 69(6):586-595), and has been linked to Nav1.7 mutations that severely impair fast-inactivation (Fertleman et al (2006) Neuron 52(5):767-774).

Small Fiber Neuropathy (SFN), which involves thinly myelinated and unmyelinated peripheral nerve fibers (Lacomis D: (2002) Muscle Nerve 26(2):173-188; Lauria G (2005) Curr Opin Neurol 18(5):591-597), presents a clinical picture that is characteristically dominated by neuropathic pain and autonomic symptoms (Stewart et al (1992) Muscle Nerve 15(6):661-665), together with preservation of normal strength, tendon reflexes, and vibration sense, and normal nerve conduction studies (NCS), which rule out large fiber involvement. The diagnosis of SFN can be confirmed by demonstration of reduced intraepidermal nerve fiber density (IENFD) on skin biopsy and/or abnormal quantitative sensory testing (QST) (Lauria et al (2010) Eur J Neurol 17(7):903-912, e944-909; Tesfaye et al (2010) Diabetes Care 33(10):2285-2293).

Therefore, there is a need to develop an objective test, in particular a rapid genotyping test, for the identification of the above mentioned paroxysmal pain disorders, such as trigeminal neuralgia, and also to identify patients most likely to respond to therapy.

### SUMMARY OF THE INVENTION

According to a first aspect of the invention, there is provided a method of diagnosing trigeminal neuralgia in a human subject wherein said method comprises detecting the presence of one or more genetic variations within the *CACNA* 1A gene and/or the *CACNA 1B* gene of said subject.

According to a further aspect of the disclosure there is provided a kit for diagnosing a paroxysmal pain disorder which comprises instructions to use said kit in accordance with the methods defined herein.

According to a further aspect of the disclosure, there is provided a method of treating a paroxysmal pain disorder in a human subject wherein said method comprises:
(a) detecting the presence of one or more genetic variations within the *CACNA1A* gene and/or the *CACNA 1B* gene of said subject; and
(b) administering a Nav1.7 sodium channel blocker to said patient identified as having said one or more genetic variations.

Thus, according to a further aspect of the disclosure, there is provided a method of predicting whether a patient will respond to treatment with a Nav1.7 sodium channel blocker, wherein said method comprises the steps of:
(a) obtaining a biological sample from a patient; and
(b) detecting the presence of one or more genetic variations within the *CACNA* 1A gene and/or the *CACNA 1B* gene of said subject;
such that the presence of said one or more genetic variations is indicative that a patient will respond to treatment with a Nav1.7 sodium channel blocker.

According to a further aspect of the disclosure, there is provided a Nav1.7 sodium channel blocker for use in the treatment of a paroxysmal pain disorder in a patient, characterised in that said patient has been selected for having one or more genetic variations within the *CACNA* 1A gene and/or the *CACNA1B* gene.

According to a further aspect of the disclosure, there is provided a method of treating a paroxysmal pain disorder in a patient wherein said method comprises the steps of selecting a patient having one or more genetic variations within the *CACNA1A* gene and/or the *CACNA1B* gene followed by administering a therapeutically effective amount of a Nav1.7 sodium channel blocker to said patient.

### BRIEF DESCRIPTION OF THE FIGURES

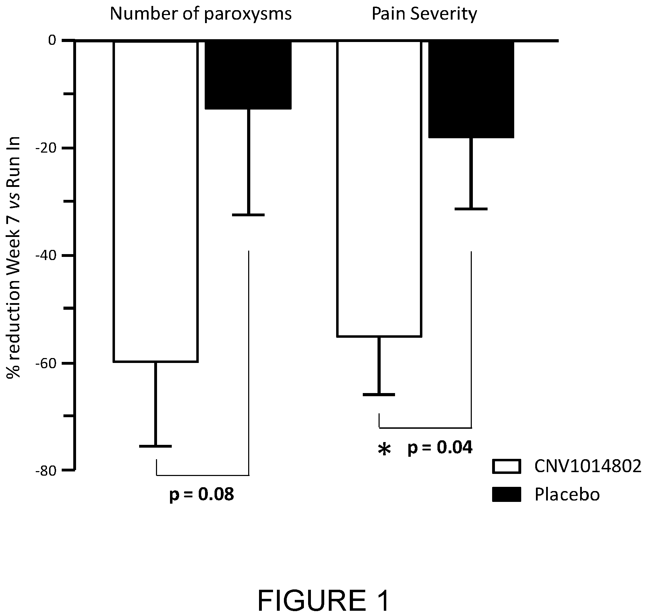

**Figure 1** describes the results of a 7-week study with CNV1014802 (a novel Nav1.7 selective state-dependent sodium channel blocker) in trigeminal neuralgia patients.

### DETAILED DESCRIPTION OF THE INVENTION

According to a first aspect of the disclosure, there is provided a method of diagnosing a paroxysmal pain disorder in a human subject wherein said method comprises detecting the presence of one or more genetic variations within the *CACNA1A* gene and/or the *CACNA1B* gene of said subject.

The study described herein has surprisingly identified a series of genetic variations within the *CACNA1A* and *CACNA1B* genes in trigeminal neuralgia patients when compared with wild type sequences. These mutants therefore have great utility in the diagnosis of patients having or likely to suffer from trigeminal neuralgia.

References herein to "paroxysmal pain disorders" refer to pain disorders which are characterised by attacks of pain. In one embodiment, the paroxysmal pain disorder is selected from: trigeminal neuralgia (TN), inherited erythromelalgia (IEM), paroxysmal extreme pain disorder (PEPD) and small fiber neuropathy (SFN). In a further embodiment, the paroxysmal pain disorder is selected from trigeminal neuralgia.

References herein to *"CACNA1A* gene" refer to the calcium channel, voltage-dependent, P/Q type, alpha 1A subunit gene (also known as Cav2.1). The *CACNA1A* gene belongs to a family of genes that code for the pore forming alpha subunits of calcium channels. These channels, which transport positively charged calcium atoms (calcium ions) across cell membranes, play a key role in a cell's ability to generate and transmit electrical signals. Calcium ions are involved in many different cellular functions, including cell-to-cell communication, the tensing of muscle fibers (muscle contraction), and the regulation of certain genes.

The *CACNA1A* gene provides instructions for making one part (the alpha-1 subunit) of a calcium channel called Cav2.1. This subunit forms the hole (pore) through which calcium ions can flow. Cav2.1 channels play an essential role in communication between nerve cells (neurons) in the brain and spinal cord. These channels help control the release of neurotransmitters, which are chemicals that relay signals from one neuron to another. It is believed that Cav2.1 channels are also involved in the survival of neurons and the ability of these cells to change and adapt over time (plasticity).

Near one end of the *CACNA1A* gene, a segment of three DNA building blocks (nucleotides) is repeated multiple times. This sequence, which is written as CAG, is called a triplet or trinucleotide repeat. In most people, the number of CAG repeats in this gene ranges from 4 to 18.

The *CACNA1A* gene is located on the short (p) arm of chromosome 19 at position 13 (19p13). More precisely, the molecular location of the *CACNA1A* gene is on chromosome 19: base pairs 13,206,441 to 13,506,459.

*CACNA1A* has been previously linked with episodic ataxia, familial hemiplegic migraine, spinocerebellar ataxia type 6 and sporadic hemiplegic migraine, however, no such link has been specifically provided between *CACNA1A* and paroxysmal pain disorders, such as trigeminal neuralgia.

References herein to *"CACNA1B* gene" refer to the calcium channel, voltage-dependent, N type, alpha 1B subunit gene (also known as Cav2.2). The protein encoded by this gene is the pore-forming subunit of an N-type voltage-dependent calcium channel, which controls neurotransmitter release from neurons. The encoded protein forms a complex with alpha-2, beta, and delta subunits to form the high-voltage activated channel. This channel is sensitive to omega-conotoxin-GVIA and omega-agatoxin-IIIA but insensitive to dihydropyridines. Two transcript variants encoding different isoforms have been found for this gene.

The *CACNA1B* gene is located on the long (q) arm of chromosome 9 at position 34 (9q34). More precisely, the molecular location of the *CACNA 1B* gene is on chromosome 9: base pairs 140,772,241 to 141,019,076.

Diseases associated with CACNA1B include krukenberg carcinoma, and morvan's fibrillary chorea, and among its related super-pathways are synaptic vesicle pathway and circadian entrainment, however, no such link has been specifically provided between CACNA1B and paroxysmal pain disorders, such as trigeminal neuralgia.

References herein to "genetic variation" refer to a difference or variation between the genetic code or sequence of a human subject when compared with a wild-type or native genetic code or sequence. It will be appreciated that the genetic variations include any variation in the wild type or native genetic code of the genome from said human subject under analysis. Examples of such genetic variations include: mutations (e.g. point mutations), insertions, substitutions, deletions, frame shifts, single nucleotide polymorphisms (SNPs), haplotypes, chromosome abnormalities, copy number variation (CNV), epigenetics and DNA inversions. In one embodiment, the genetic variations are selected from substitutions, insertions, deletions and frame shifts.

References herein to the term "single-nucleotide polymorphism (SNP)" is intended to refer to DNA sequence variation occurring when a single nucleotide in the genome (or other shared sequence) differs between members of a species or between paired chromosomes in an individual.

References herein to the term "haplotype" is intended to refer to a set of genetic markers that are inherited together as a consequence of their chromosomal co-localization. Haplotype may refer to as few as two genetic variants or to an entire chromosome depending on the number of recombination events that have occurred between a given set of variants.

In one embodiment, the genetic variations are detected within the *CACNA1A* gene. In a further embodiment, the genetic variations are within the *CACNA1A* gene and are between 13,300,000 bp and 13,450,000 bp on chromosome 19, such as between 13,318,000 bp and 13,412,000 bp on chromosome 19.

In a yet further embodiment, the genetic variations are within the *CACNA1A* gene and are selected from one or more of:
(a) a substitution and deletion mutation at 13,318,671 bp;
(b) a substitution and deletion mutation at 13,318,672 bp;
(c) a substitution and deletion mutation at 13,318,707 bp;
(d) a frame shift mutation at 13,318,709 bp;
(e) a substitution mutation at 13,318,811 bp;
(f) an insertion mutation at 13,319,691 bp;
(g) an insertion mutation at 13,319,712 bp;
(h) a substitution mutation at 13,397,560 bp;
(i) a substitution mutation at 13,409,407 bp; and
(j) a substitution mutation at 13,411,451 bp.
This embodiment relates to the novel and rare (<10% general population) mutations identified within the *CACNA1A* gene in the study defined herein.

In a yet further embodiment, the genetic variations are within the *CACNA1A* gene and are selected from one or more of:
(a) a substitution and deletion mutation at 13,318,671 bp;
(b) a substitution and deletion mutation at 13,318,672 bp;
(c) a substitution and deletion mutation at 13,318,707 bp;
(d) a frame shift mutation at 13,318,709 bp;
(f) an insertion mutation at 13,319,691 bp; and
(g) an insertion mutation at 13,319,712 bp.
This embodiment relates to the novel mutations identified within the *CACNA1A* gene in the study defined herein.

In a still yet further embodiment, the genetic variations are within the *CACNA1A* gene and are selected from:
(b) a substitution and deletion mutation at 13,318,672 bp.
This embodiment relates to the novel mutation identified within the *CACNA1A* gene in 62% of TGN patients in the study defined herein.

The substitution and deletion mutation at 13,318,671 bp described in (a) above is considered a novel mutation because this mutation has not been previously identified. In one embodiment, the substitution and deletion mutation at 13,318,671 bp described in (a) above comprises substitution and deletion of the sequence: CAG CAG CAG CAG CAG CAG GCG (SEQ ID NO: 1) with the sequence: CCG. For example, the first six codons starting at position 13,318,671 bp are deleted and in the seventh codon the first nucleotide of the codon has a G substituted by a C. At the protein level, this substitution and deletion mutation results in a QQQQQQA(SEQ ID NO: 2)2320P mutation.

The substitution and deletion mutation at 13,318,672 bp described in (b) above is considered a novel mutation because this mutation has not been previously identified. In one embodiment, the substitution and deletion mutation at 13,318,672 bp described in (b) above comprises substitution and deletion of the sequence: CCG CAG CAG with the sequence: CCG. For example, the first two codons starting at position 13,318,672 bp are deleted and in the third codon the second nucleotide of the codon has an A substituted by a C. At the protein level, this deletion mutation results in a PQQ2312P mutation.

The substitution and deletion mutation at 13,318,707 bp described in (c) above is considered a novel mutation because this mutation has not been previously identified. In one embodiment, the substitution and deletion mutation at 13,318,707 bp described in (c) above comprises substitution and deletion of the sequence: CCC GCA GCA with the sequence: CCA. For example, the first two codons starting at position 13,318,707 bp are deleted and in the third codon the first nucleotide of the codon has a G substituted by a C. At the protein level, this substitution and deletion mutation results in a PAA2316P mutation. The mutation described in (c) above is considered a preferred mutation for monitoring because the data shown herein indicates that this mutation was surprisingly observed in 62% of TGN patients.

The frame shift mutation at 13,318,709 bp described in (d) above is considered a novel mutation because this mutation has not been previously identified. In one embodiment, the frame shift mutation at 13,318,709 bp described in (d) above comprises substitution of the sequence: CTGCG with the sequence: C. For example, the second to fifth nucleotides after position 13,318,709 bp are deleted which causes a frameshift. At the protein level, this frame shift mutation results in coding of different amino acids after residue number 2312.

The substitution mutation at 13,318,811 bp described in (e) above is considered a rare mutation because this mutation has only been identified at a frequency of 0.02. This mutation has previously been assigned the identification number: rs147221323. In one embodiment, the substitution mutation at 13,318,811 bp described in (e) above comprises substitution of the sequence: GCG with the sequence: ACG. For example, the first nucleotide starting at position 13,318,811 bp has a G substituted by an A. At the protein level, this substitution mutation results in a A2284T mutation.

The insertion mutation at 13,319,691 bp described in (f) above is considered a novel mutation because this mutation has not been previously identified. In one embodiment, the insertion mutation at 13,319,691 bp described in (f) above comprises insertion of a CAC codon after the CAT codon at position 13,319,691 bp. At the protein level, this substitution mutation results in a H2219HH mutation.

The insertion mutation at 13,319,712 bp described in (g) above is considered a novel mutation because this mutation has not been previously identified. In one embodiment, the insertion mutation at 13,319,712 bp described in (g) above comprises insertion of a CCC codon after the CAC codon at position 13,319,712 bp. At the protein level, this substitution mutation results in a H2212HP mutation.

The substitution mutation at 13,397,560 bp described in (h) above is considered a rare mutation because this mutation has only been identified at a frequency of 0.07. This mutation has previously been assigned the identification number: rs16027. In one embodiment, the substitution mutation at 13,397,560 bp described in (h) above comprises substitution of the sequence: GGC with the sequence: AGC. For example, the first nucleotide starting at position 13,397,560 bp has a G substituted by an A. At the protein level, this substitution mutation results in a G1104S mutation.

The substitution mutation at 13,409,407 bp described in (i) above is considered a rare mutation because this mutation has only been identified at a frequency of <0.01. This mutation has previously been assigned the identification number: rs16024. In one embodiment, the substitution mutation at 13,409,407 bp described in (i) above comprises substitution of the sequence: GAG with the sequence: AAG. For example, the first nucleotide starting at position 13,409,407 bp has a G substituted by an A. At the protein level, this substitution mutation results in a E1014K mutation.

The substitution mutation at 13,411,451 bp described in (j) above is considered a rare mutation because this mutation has only been identified at a frequency of 0.01. This mutation has previously been assigned the identification number: rs16019. In one embodiment, the substitution mutation at 13,411,451 bp described in (j) above comprises substitution of the sequence: GAA with the sequence: GCA. For example, the second nucleotide after position 13,411,451 bp has an A substituted by a C. At the protein level, this substitution mutation results in a E731A mutation.

In one embodiment, the genetic variations are detected within the CACNA1B gene. In a further embodiment, the genetic variations are within the CACNA1B gene and are between 140,750,000 bp and 140,950,000 bp on chromosome 9, such as between 140,772,000 bp and 141,000,000 bp on chromosome 9.

In a yet further embodiment, the genetic variations are within the CACNA1B gene and are selected from one or more of:
(a) a substitution mutation at 140,772,434 bp;
(b) a substitution mutation at 140,772,440 bp;
(c) a substitution mutation at 140,772,477 bp;
(d) a substitution mutation at 140,777,306 bp; and
(e) a substitution mutation at 140,918,181 bp.
This embodiment relates to the novel and rare (<10% general population) mutations identified within the *CACNA1B* gene in the study defined herein.

In a still yet further embodiment, the genetic variations are within the *CACNA1B* gene and are selected from:
(b) a substitution mutation at 140,772,440 bp.
This embodiment relates to the sole novel mutation identified within the *CACNA1B* gene in the study defined herein.

The substitution mutation at 140,772,434 bp described in (a) above is considered a rare mutation because this mutation has only been identified at a frequency of 0.062. This mutation has previously been assigned the identification number: rs187204220. In one embodiment, the substitution mutation at 140,772,434 bp described in (a) above comprises substitution of the sequence: GGC with the sequence: AGC. For example, the second nucleotide after position 140,772,434 bp has a G substituted by an A. At the protein level, this substitution mutation results in a G17S mutation.

The substitution mutation at 140,772,440 bp described in (b) above is considered a novel mutation because this mutation has not been previously identified. In one embodiment, the substitution mutation at 140,772,440 bp described in (b) above comprises substitution of the sequence: GAG with the sequence: AAG. For example, the first nucleotide at position 140,772,440 bp has a G substituted by an A. At the protein level, this substitution mutation results in a E19K mutation.

The substitution mutation at 140,772,477 bp described in (c) above is considered a rare mutation because this mutation has only been identified at a frequency of <0.001. This mutation has previously been assigned the identification number: rs201253748. In one embodiment, the substitution mutation at 140,772,477 bp described in (c) above comprises substitution of the sequence: GGC with the sequence: GTC. For example, the second nucleotide after position 140,772,477 bp has a G substituted by a T. At the protein level, this substitution mutation results in a G31V mutation.

The substitution mutation at 140,777,306 bp described in (d) above is considered a rare mutation because this mutation has only been identified at a frequency of <0.001. This mutation has previously been assigned the identification number: rs4422842. In one embodiment, the substitution mutation at 140,777,306 bp described in (d) above comprises substitution of the sequence: AAC with the sequence: AAG. For example, the third nucleotide after position 140,777,306 bp has a C substituted by a G. At the protein level, this substitution mutation results in a N167K mutation.

The substitution mutation at 140,918,181 bp described in (e) above is considered a rare mutation because this mutation has only been identified at a frequency of <0.001. This mutation has previously been assigned the identification number: rs11137342. In one embodiment, the substitution mutation at 140,918,181 bp described in (e) above comprises substitution of the sequence: ACC with the sequence: GCC. For example, the first nucleotide at position 140,918,181 bp has an A substituted by a G. At the protein level, this substitution mutation results in a T996A mutation.

Data obtained from the study described herein identified that 32% of trigeminal neuralgia patients had more than one of the above mentioned mutations in the *CACNA1A* gene and 41% had more than one of the above mentioned mutations in the *CACNA1B.* Thus, in one embodiment, said diagnostic method comprises more than one genetic variation (e.g. two, three or more genetic variations).

The methods of the disclosure may be used to detect genetic variations using a biological sample obtained from the human subject. Thus, in one embodiment, the method initially comprises the step of obtaining a biological sample from the human subject.

The nucleic acid may be isolated from the sample according to any methods well known to those of skill in the art. Examples include tissue samples or any cell-containing or a cellular biomaterial (i.e. a bodily fluid or hair sample). Biological samples may be obtained by standard procedures and may be used immediately or stored, under conditions appropriate for the type of biological sample, for later use.

Methods of obtaining biological samples are well known to those of skill in the art and include, but are not limited to, aspirations, tissue sections, drawing of blood or other fluids, surgical or needle biopsies, and the like. Examples of suitable biological samples include: whole blood, blood serum, plasma, urine, saliva, or other bodily fluid (stool, tear fluid, synovial fluid, sputum), hair, cerebrospinal fluid (CSF), or an extract or purification therefrom, or dilution thereof. Biological samples also include tissue homogenates, tissue sections and biopsy specimens from a live subject. The samples can be prepared, for example where appropriate diluted or concentrated, and stored in the usual manner. In one embodiment, the biological sample is blood or serum. In a further embodiment, the biological sample is blood. If necessary, the sample may be collected or concentrated by centrifugation and the like.

The cells of the sample may be subjected to lysis, such as by treatments with enzymes, heat, surfactants, ultrasonication, or a combination thereof. The lysis treatment is performed in order to obtain a sufficient amount of nucleic acid derived from the cells of the human subject to detect using polymerase chain reaction.

Methods of plasma and serum preparation are well known in the art. Either "fresh" blood plasma or serum, or frozen (stored) and subsequently thawed plasma or serum may be used. Frozen (stored) plasma or serum should optimally be maintained at storage conditions of -20 to -70°C until thawed and used. "Fresh" plasma or serum should be refrigerated or maintained on ice until used, with nucleic acid (e.g., RNA, DNA or total nucleic acid) extraction being performed as soon as possible. Exemplary methods are described below.

Blood can be drawn by standard methods into a collection tube, typically siliconized glass, either without anticoagulant for preparation of serum, or with EDTA, sodium citrate, heparin, or similar anticoagulants for preparation of plasma. If preparing plasma or serum for storage, although not an absolute requirement, plasma or serum is first fractionated from whole blood prior to being frozen. This reduces the burden of extraneous intracellular RNA released from lysis of frozen and thawed cells which might reduce the sensitivity of the amplification assay or interfere with the amplification assay through release of inhibitors to PCR such as porphyrins and hematin. "Fresh" plasma or serum may be fractionated from whole blood by centrifugation, using gentle centrifugation at 300-800 times gravity for five to ten minutes, or fractionated by other standard methods. High centrifugation rates capable of fractionating out apoptotic bodies should be avoided.

It will be appreciated that the step of detecting the one or more genetic variations within the regions defined herein will comprise any suitable technique for genetic analysis.

The volume of plasma or serum used in the extraction may vary, but volumes of 1 to 100 ml of plasma or serum are usually sufficient.

Various methods of extraction are suitable for isolating the nucleic acid. Suitable methods include phenol and chloroform extraction. See Maniatis et al., Molecular Cloning, A Laboratory Manual, 2d, Cold Spring Harbor Laboratory Press, page 16.54 (1989).

Numerous commercial kits also yield suitable DNA and RNA including, but not limited to, QIAamp™ mini blood kit, Agencourt Genfind™, Roche Cobas® Roche MagNA Pure® or phenol: chloroform extraction using Eppendorf Phase Lock Gels®, and the NucliSens extraction kit (Biomerieux, Marcy I'Etoile, France). In other methods, mRNA may be extracted from blood/bone marrow samples using MagNA Pure LC mRNA HS kit and Mag NA Pure LC Instrument (Roche Diagnostics Corporation, Roche Applied Science, Indianapolis, IN).

Nucleic acid extracted from tissues, cells, plasma, serum or hair can be amplified using nucleic acid amplification techniques well known in the art. Many of these amplification methods can also be used to detect the presence of genetic variations simply by designing oligonucleotide primers or probes to interact with or hybridize to a particular target sequence in a specific manner. By way of example, but not by way of limitation, these techniques can include the polymerase chain reaction (PCR), reverse transcriptase polymerase chain reaction (RT-PCR), nested PCR, ligase chain reaction. See Abravaya, K., et al, Nucleic Acids Research, 23:675-682, (1995), branched DNA signal amplification, Urdea, M. S., et al, AIDS, 7 (suppl 2):S11-S 14, (1993), amplifiable RNA reporters, Q-beta replication, transcription-based amplification, boomerang DNA amplification, strand displacement activation, cycling probe technology, isothermal nucleic acid sequence based amplification (NASBA). See Kievits, T. et al, J Virological Methods, 35:273-286, (1991), Invader Technology, or other sequence replication assays or signal amplification assays. These methods of amplification each described briefly below and are well-known in the art.

Some methods employ reverse transcription of RNA to cDNA. Various reverse transcriptases may be used, including, but not limited to, MMLV RT, RNase H mutants of MMLV RT such as Superscript and Superscript II (Life Technologies, GIBCO BRL, Gaithersburg, Md.), AMV RT, and thermostable reverse transcriptase from Thermus Thermophilus. For example, one method, but not the only method, which may be used to convert RNA extracted from plasma or serum to cDNA is the protocol adapted from the Superscript II Preamplification system (Life Technologies, GIBCO BRL, Gaithersburg, Md.; catalog no. 18089-011), as described by Rashtchian, A., PCR Methods Applic, 4:S83-S91, (1994).

A variety of amplification enzymes are well known in the art and include, for example, DNA polymerase, RNA polymerase, reverse transcriptase, Q-beta replicase, thermostable DNA and RNA polymerases. Because these and other amplification reactions are catalyzed by enzymes, in a single step assay the nucleic acid releasing reagents and the detection reagents should not be potential inhibitors of amplification enzymes if the ultimate detection is to be amplification based. Amplification methods suitable for use with the present methods include, for example, strand displacement amplification, rolling circle amplification, primer extension preamplification, or degenerate oligonucleotide PCR (DOP).

In one embodiment, PCR is used to amplify a target or marker sequence of interest. The person skilled in the art is capable of designing and preparing primers that are appropriate for amplifying a target sequence. The length of the amplification primers depends on several factors including the nucleotide sequence identity and the temperature at which these nucleic acids are hybridized or used during *in vitro* nucleic acid amplification. The considerations necessary to determine a preferred length for an amplification primer of a particular sequence identity are well-known to the person skilled in the art. For example, the length of a short nucleic acid or oligonucleotide can relate to its hybridization specificity or selectivity.

For analyzing SNPs and other variant nucleic acids, it may be appropriate to use oligonucleotides specific for alternative alleles. Such oligonucleotides which detect single nucleotide variations in target sequences may be referred to by such terms as "allele-specific probes", or "allele-specific primers". The design and use of allele-specific probes for analyzing polymorphisms is described in, e.g., Mutation Detection A Practical Approach, ed. Cotton et al. Oxford University Press, 1998; Saiki et al, Nature, 324: 163-166 (1986); Dattagupta, EP235,726; and Saiki, WO 89/11548. In one embodiment, a probe or primer may be designed to hybridize to a segment of target DNA such that the SNP aligns with either the 5' most end or the 3' most end of the probe or primer.

In some embodiments, the amplification may include a labeled primer, thereby allowing detection of the amplification product of that primer. In particular embodiments, the amplification may include a multiplicity of labeled primers; typically, such primers are distinguishably labeled, allowing the simultaneous detection of multiple amplification products.

In one type of PCR-based assay, an allele-specific primer hybridizes to a region on a target nucleic acid molecule that overlaps a SNP position and only primes amplification of an allelic form to which the primer exhibits perfect complementarity (Gibbs, 1989, Nucleic Acid Res., 17:2427-2448). Typically, the primer's 3'-most nucleotide is aligned with and complementary to the SNP position of the target nucleic acid molecule. This primer is used in conjunction with a second primer that hybridizes at a distal site. Amplification proceeds from the two primers, producing a detectable product that indicates which allelic form is present in the test sample. A control is usually performed with a second pair of primers, one of which shows a single base mismatch at the polymorphic site and the other of which exhibits perfect complementarity to a distal site. The single-base mismatch prevents amplification or substantially reduces amplification efficiency, so that either no detectable product is formed or it is formed in lower amounts or at a slower pace. The method generally works most effectively when the mismatch is at the 3'-most position of the oligonucleotide (i.e., the 3'-most position of the oligonucleotide aligns with the target SNP position) because this position is most destabilizing to elongation from the primer (see, e.g., WO 93/22456).

In a specific embodiment, a primer contains a sequence substantially complementary to a segment of a target SNP-containing nucleic acid molecule except that the primer has a mismatched nucleotide in one of the three nucleotide positions at the 3'-most end of the primer, such that the mismatched nucleotide does not base pair with a particular allele at the SNP site. In one embodiment, the mismatched nucleotide in the primer is the second from the last nucleotide at the 3'-most position of the primer. In another embodiment, the mismatched nucleotide in the primer is the last nucleotide at the 3'-most position of the primer.

In one embodiment, primer or probe is labeled with a fluorogenic reporter dye that emits a detectable signal. While a suitable reporter dye is a fluorescent dye, any reporter dye that can be attached to a detection reagent such as an oligonucleotide probe or primer is suitable for use in the invention. Such dyes include, but are not limited to, Acridine, AMCA, BODIPY, Cascade Blue, Cy2, Cy3, Cy5, Cy7, Dabcyl, Edans, Eosin, Erythrosin, Fluorescein, 6-Fam, Tet, Joe, Hex, Oregon Green, Rhodamine, Rhodol Green, Tamra, Rox, and Texas Red.

It will be appreciated that the disclosure extends to reagents that do not contain (or that are complementary to) a SNP nucleotide identified herein but that are used to assay one or more SNPs disclosed herein. For example, primers that flank, but do not hybridize directly to a target SNP position provided herein are useful in primer extension reactions in which the primers hybridize to a region adjacent to the target SNP position (i.e., within one or more nucleotides from the target SNP site). During the primer extension reaction, a primer is typically not able to extend past a target SNP site if a particular nucleotide (allele) is present at that target SNP site, and the primer extension product can readily be detected in order to determine which SNP allele is present at the target SNP site. For example, particular ddNTPs are typically used in the primer extension reaction to terminate primer extension once a ddNTP is incorporated into the extension product. Thus, reagents that bind to a nucleic acid molecule in a region adjacent to a SNP site, even though the bound sequences do not necessarily include the SNP site itself, are also encompassed by the disclosure.

Variant nucleic acids may be amplified prior to detection or may be detected directly during an amplification step (i.e., "real-time" methods). In some embodiments, the target sequence is amplified and the resulting amplicon is detected by electrophoresis. In some embodiments, the specific mutation or variant is detected by sequencing the amplified nucleic acid. In some embodiments, the target sequence is amplified using a labeled primer such that the resulting amplicon is detectably labeled. In some embodiments, the primer is fluorescently labeled.

In one embodiment, detection of a variant nucleic acid, such as a SNP, is performed using the TaqMan® assay, which is also known as the 5' nuclease assay (U.S. Pat. Nos. 5,210,015 and 5,538,848) or Molecular Beacon probe (U.S. Pat. Nos. 5,118,801 and 5,312,728), or other stemless or linear beacon probe (Livak et al, 1995, PCR Method AppL, 4:357-362; Tyagi et al, 1996, Nature Biotechnology, 14:303-308; Nazarenko et al, 1997, Nucl. Acids Res., 25:2516-2521; U.S. Pat. Nos. 5,866,336 and 6,117,635). The TaqMan® assay detects the accumulation of a specific amplified product during PCR. The TaqMan® assay utilizes an oligonucleotide probe labeled with a fluorescent reporter dye and a quencher dye. The reporter dye is excited by irradiation at an appropriate wavelength, it transfers energy to the quencher dye in the same probe via a process called fluorescence resonance energy transfer (FRET). When attached to the probe, the excited reporter dye does not emit a signal. The proximity of the quencher dye to the reporter dye in the intact probe maintains a reduced fluorescence for the reporter. The reporter dye and quencher dye may be at the 5' most and the 3' most ends, respectively or vice versa. Alternatively, the reporter dye may be at the 5 ' or 3' most end while the quencher dye is attached to an internal nucleotide, or vice versa. In yet another embodiment, both the reporter and the quencher may be attached to internal nucleotides at a distance from each other such that fluorescence of the reporter is reduced.

During PCR, the 5' nuclease activity of DNA polymerase cleaves the probe, thereby separating the reporter dye and the quencher dye and resulting in increased fluorescence of the reporter. Accumulation of PCR product is detected directly by monitoring the increase in fluorescence of the reporter dye. The DNA polymerase cleaves the probe between the reporter dye and the quencher dye only if the probe hybridizes to the target SNP- containing template which is amplified during PCR, and the probe is designed to hybridize to the target SNP site only if a particular SNP allele is present.

TaqMan® primer and probe sequences can readily be determined using the variant and associated nucleic acid sequence information provided herein. A number of computer programs, such as Primer Express (Applied Biosystems, Foster City, Calif.), can be used to rapidly obtain optimal primer/probe sets. It will be apparent to one of skill in the art that such primers and probes for detecting the genetic variations of the disclosure are useful in diagnosis of paroxysmal pain disorders, and can be readily incorporated into a kit format.

The disclosure also includes modifications of the TaqMan® assay well known in the art such as the use of Molecular Beacon probes (U.S. Pat. Nos. 5,118,801 and 5,312,728) and other variant formats (U.S. Pat. Nos. 5,866,336 and 6,117,635).

Other methods of probe hybridization detected in real time can be used for detecting amplification of a target or marker sequence flanking a tandem repeat region. For example, the commercially available MGB Eclipse™ probes (Epoch Biosciences), which do not rely on a probe degradation can be used. MGB Eclipse™ probes work by a hybridization-triggered fluorescence mechanism. MGB Eclipse™ probes have the Eclipse™ Dark Quencher and the MGB positioned at the 5'-end of the probe. The fluorophore is located on the 3'-end of the probe. When the probe is in solution and not hybridized, the three dimensional conformation brings the quencher into close proximity of the fluorophore, and the fluorescence is quenched. However, when the probe anneals to a target or marker sequence, the probe is unfolded, the quencher is moved from the fluorophore, and the resultant fluorescence can be detected.

Oligonucleotide probes can be designed which are between about 10 and about 100 nucleotides in length and hybridize to the amplified region. Oligonucleotides probes are preferably 12 to 70 nucleotides; more preferably 15-60 nucleotides in length; and most preferably 15-25 nucleotides in length. The probe may be labeled. Amplified fragments may be detected using standard gel electrophoresis methods. For example, in preferred embodiments, amplified fractions are separated on an agarose gel and stained with ethidium bromide by methods known in the art to detect amplified fragments.

Another suitable detection methodology involves the design and use of bipartite primer/probe combinations such as Scorpion™ probes. These probes perform sequence-specific priming and PCR product detection is achieved using a single molecule. Scorpion™ probes comprise a 3' primer with a 5' extended probe tail comprising a hairpin structure which possesses a fluorophore/quencher pair. The probe tail is "protected" from replication in the 5' to 3' direction by the inclusion of hexethlyene glycol (HEG) which blocks the polymerase from replicating the probe. The fluorophore is attached to the 5' end and is quenched by a moiety coupled to the 3' end. After extension of the Scorpion™ primer, the specific probe sequence is able to bind to its complement within the extended amplicon thus opening up the hairpin loop. This prevents the fluorescence from being quenched and a signal is observed. A specific target is amplified by the reverse primer and the primer portion of the Scorpion™, resulting in an extension product. A fluorescent signal is generated due to the separation of the fluorophore from the quencher resulting from the binding of the probe element of the Scorpion™ to the extension product. Such probes are described in Whitcombe et al, Nature Biotech 17: 804-807 (1999). Determining Prognosis

According to a further aspect of the disclosure there is provided a kit for diagnosing a paroxysmal pain disorder which comprises instructions to use said kit in accordance with the methods defined herein.

The kits may be prepared for practicing the methods described herein. Typically, the kits include at least one component or a packaged combination of components useful for practicing the method of the invention. The kits may include some or all of the components necessary to practice method of the invention. Typically, the kits include at least one probe specific for the one or more regions defined herein in at least one container. These components may include, *inter alia,* nucleic acid probes, nucleic acid primers for amplification of the one or more regions defined herein, buffers, instructions for use, and the like.

It will be appreciated that once an individual has been identified as likely to suffer from or suffering from a paroxysmal pain disorder, said individual will then be prescribed suitable treatment, such as a Nav1.7 sodium channel blocker.

Thus, according to a further aspect of the disclosure, there is provided a method of treating a paroxysmal pain disorder in a human subject wherein said method comprises:
(a) detecting the presence of one or more genetic variations within the *CACNA1A* gene and/or the *CACNA1B* gene of said subject; and
(b) administering a Nav1.7 sodium channel blocker to said patient identified as having said one or more genetic variations.

Examples of suitable Nav1.7 sodium channel blockers are described in WO 2007/042239, WO 2007/042250, WO 2007/042240, WO 2013/179049, WO 2013/093497, WO 2013/093496, WO 2013/175206 and WO 2013/175205. In one embodiment, the Nav1.7 sodium channel blocker is selected from a compound described in WO 2007/042250. In a further embodiment, the Nav1.7 sodium channel blocker is selected from a compound described in WO 2007/042239. In a yet further embodiment, the Nav1.7 sodium channel blocker is (5R)-5-(4-{[(2-fluorophenyl)methyl]oxy}phenyl)-L-prolinamide or a pharmaceutically acceptable salt or solvate thereof as shown in formula (I):

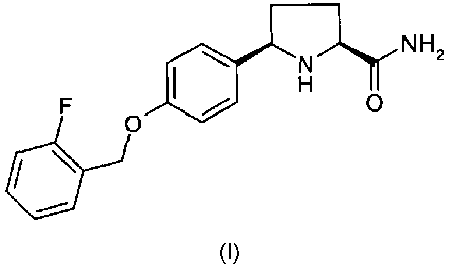

In a yet further embodiment, the Nav1.7 sodium channel blocker is a Nav1.7 selective state-dependent sodium channel blocker, such as (5R)-5-(4-{[(2-fluorophenyl)methyl]oxy}phenyl)-L-prolinamide hydrochloride salt (CNV1014802).

As discussed hereinbefore, current treatment for paroxysmal pain disorders is associated with poor tolerability and results in sub-optimal pain control. There is therefore a great need to identify individuals most likely to respond to such therapy.

Thus, according to a further aspect of the disclosure, there is provided a method of predicting whether a patient will respond to treatment with a Nav1.7 sodium channel blocker, wherein said method comprises the steps of:
(a) obtaining a biological sample from a patient; and
(b) detecting the presence of one or more genetic variations within the *CACNA* 1A gene and/or the *CACNA1B* gene of said subject;
such that the presence of said one or more genetic variations is indicative that a patient will respond to treatment with a Nav1.7 sodium channel blocker.

Data is shown in the study herein that all patients with genetic variations within the *CACNA1A* gene and/or the *CACNA1B* gene surprisingly responded to treatment with a Nav1.7 sodium channel blocker, such as CNV1014802.

It will be appreciated that once a "responder" has been identified, the patient will then suitably be administered with a Nav1.7 sodium channel blocker.

Thus, in one embodiment, the method additionally comprises:
(c) administering a Nav1.7 sodium channel blocker to said patient identified as having said one or more genetic variations.

According to a further aspect of the disclosure, there is provided a Nav1.7 sodium channel blocker for use in the treatment of a paroxysmal pain disorder in a patient, characterised in that said patient has been selected for having one or more genetic variations within the *CACNA1A* gene and/or the *CACNA1B* gene.

According to a further aspect of the disclosure, there is provided a method of treating a paroxysmal pain disorder in a patient wherein said method comprises the steps of selecting a patient having one or more genetic variations within the *CACNA1A* gene and/or the *CACNA1B* gene followed by administering a therapeutically effective amount of a Nav1.7 sodium channel blocker to said patient.

The following studies illustrate the invention.

### Example 1: 7 Week Trial with Trigeminal Neuralgia (TGN) patients Identifying Responders to CNV1014802 Therapy

CNV1014802 is a novel Nav1.7 selective state-dependent sodium channel blocker that is being assessed in the treatment of trigeminal neuralgia. The trial used a randomized withdrawal design to assess efficacy in this rare condition and comprised a 21-day open-label phase followed by a randomized 28-day placebo-controlled phase for responders as described in Zakrzewska et al., (2013) Trials 14:402).

Patients participated in an initial open-label treatment period of 21 days of CNV1014802 using 150 mg three times daily. Responders were then randomized to 28 days of CNV1014802 150 mg three times daily or placebo. A responder at the end of the open-label period was defined as a patient with one of the following:
1) a 30% or more decrease in the total number of paroxysms over the last 7 days of the open-label phase as compared to the total number recorded in the 7-day baseline phase (day -7 to -1 prior to start of study medication, Run in); and/or
2) a 30% reduction in the mean severity of pain experienced during the paroxysm over the last 7 days of the open-label phase as compared to the total number recorded in the 7-day baseline phase (day -7 to -1 prior to start of study medication, run in); and/or
3) a Patient Global Improvement of Change rating of much improved/very much improved.

Responders were randomized to CNV1014802 or placebo, in a 1:1 ratio. During the double-blind randomized phase, patients were evaluated at each clinic visit, which occurred every 7 days to determine whether they met the failure criteria in the double-blind treatment period.

Patients were classified as treatment failure if they met one of the following criteria:
1) a 50% increase in the frequency of paroxysms compared to the final 7 days of the open-label period; and/or
2) a 50% increase in the severity of pain experienced in the paroxysms compared to the final 7 days of the open-label period; and/or
3) a Patient Global Improvement of Change rating of much worse/very much worse; and/or
4) the patient discontinues the study because of 'lack of efficacy'; and/or
5) the patient discontinues because of an adverse reaction or poor tolerability considered to be related to the study medication.

The data for the frequency of paroxysms and severity of the pain were taken from the daily diaries completed by the patients from the 7 day run-in period to the end of the double-blind period (week 7). When patients met the treatment failure criteria, they left the study and went back to their previous medication. The average number of paroxysms and pain severity collected in the final week of double-blind (week 7) were compared to the run in 7 day period using a T-test (equal variance assumed, unpaired).The Last Observation Carried Forward (LOCF) was used for patients meeting treatment failure criteria before the end of the double blind period (week 7). The results of the study are shown in Figure 1, where it can be seen that the reduction in the number of paroxysms and pain severity was superior in the CNV1014802 group compared to the placebo group (p=0.08 and p=0.04, respectively).

### Example 2: Genotyping Analysis of Trigeminal Neuralgia (TGN) Patients

Two calcium channel (Cav2.2 and Cav2.1) genes were sequenced in blood samples taken from the trigeminal neuralgia (TGN) patients indicated in the study described in Example 1 as responders in order to explore whether rare mutations (in less than 10% of the general population) in these genes are present in TGN.

| ***Channel*** | ***Target Gene*** | ***Co-ordinates*** |
|---|---|---|
| Cav2.1 | CACNA1A | GRCh37 (hg 19) NM_023035.2 |
| Cav2.2 | CACNA1B | GRCh37 (hg 19) NM_000718.3 |

The analysis was performed using next generation sequencing utilising a customised Agilent Haloplex Enrichment Panel and the Illumina MiSeq instrument to sequence the exonic regions of the candidate target genes. Genomic DNA was isolated from whole blood samples using a Qiagen DNA column-based purification kit. Quality and quantification checks were performed prior to PCR to ensure high quality DNA. Bioinformatics analysis of the sequencing data and detection of mutations in target genes was performed using GATK.

The incidence of mutations was evaluated in a matching population using the 1000G database.

37 samples from TGN patients were collected and the results are shown in Table 1 and may be summarised as follows:
10 non-synonymous (coding) variants of *CACNA1A* (alpha subunit of P/Q-type calcium channel) were identified across all patients: 4 rare (= in less than 10% of general population) and 6 novel. 86% of TGN patients had a rare or novel P/Q mutation and 32% had more than one. One novel mutation (the substitution and deletion mutation at 13,318,707 bp of *CACNA1A)* which results in a 2 amino acid deletion in the C-terminus of the P/Q channel is found in 62% of TGN patients.
5 non-synonymous (coding) variants of CACNA1B (alpha subunit of N-type calcium channel) were identified across all patients: 4 rare (= in less than 10% of general population) and 1 novel. 24% of TGN patients had a rare or novel N mutation. 41% of TGN patients had more than 1 N and/or P/Q mutation. Variants were deletion or miss sense, homo- or heterozygote.

The results of this study have identified a set of mutations present in responders to TGN therapy which are not found in normal patients. These mutations will be of utility not only in the diagnosis of TGN but to identify patients likely to respond to treatment with a Nav1.7 sodium channel blocker such as CNV1014802.

**Table 1: CACNA1A and CACNA1B Genotyping Results**

| **Chromosome** | **Nucleotide Position** | **Mutation ID** | **1000G Frequency** | **Gene** | **Mutation Type** | **Nucleotide Change** | **Amino Acid Change** |
|---|---|---|---|---|---|---|---|
| chr9 | 140772434 | rs187204220 | 0.062 | CACNA1B | MISSENSE | Ggc/Agc | G17S |
| chr9 | 140772440 | . | novel | CACNA1B | MISSENSE | Gag/Aag | E19K |
| chr9 | 140772477 | rs201253748 | <0.001 | CACNA1B | MISSENSE | gGc/gTc | G31V |
| chr9 | 140777306 | rs4422842 | <0.001 | CACNA1B | MISSENSE | aaC/aaG | N167K |
| chr9 | 140918181 | rs11137342 | <0.001 | CACNA1B | MISSENSE | Acc/Gcc | T996A |
| chr19 | 13318671 | . | novel | CACNA1A | DELETION | cagcagcagcagcagcaggcg (SEQ ID NO: 1)/ccg | QQQQQQA(SEQ ID NO: 2)2320P |
| chr19 | 13318672 | . | novel | CACNA1A | DELETION | ccgcagcag/ccg | PQQ2312P |
| chr19 | 13318707 | . | novel | CACNA1A | DELETION | cccgcagca/cca | PAA2316P |
| chr19 | 13318709 | . | novel | CACNA1A | FRAME SHIFT | CTGCG/C | -2312 |
| chr19 | 13318811 | rs147221323 | 0.02 | CACNA1A | MISSENSE | Gcg/Acg | A2284T |
| chr19 | 13319691 | . | novel | CACNA1A | INSERTION | cat/catCAC | H2219HH |
| chr19 | 13319712 | . | novel | CACNA1A | INSERTION | cac/caCCCc | H2212HP |
| chr19 | 13397560 | rs16027 | 0.07 | CACNA1A | MISSENSE | Ggc/Agc | G1104S |
| chr19 | 13409407 | rs16024 | <0.01 | CACNA1A | MISSENSE | Gag/Aag | E1014K |
| chr19 | 13411451 | rs16019 | 0.01 | CACNA1A | MISSENSE | gAa/gCa | E731A |

## Claims

1. A method of diagnosing trigeminal neuralgia in a human subject wherein said method comprises detecting the presence of one or more genetic variations within the *CACNA1A* gene and/or the *CACNA1B* gene of said subject.

2. The method as defined in claim 1, wherein the genetic variations include: mutations (e.g. point mutations), insertions, substitutions, deletions, frame shifts, single nucleotide polymorphisms (SNPs), haplotypes, chromosome abnormalities, copy number variation (CNV), epigenetics and DNA inversions.

3. The method as defined in claim 2, wherein the genetic variations are selected from substitutions, insertions, deletions and frame shifts.

4. The method as defined in any one of claims 1 to 3, wherein the genetic variations are detected within the *CACNA1A* gene and the genetic variations are between 13,300,000 bp and 13,450,000 bp on chromosome 19.

5. The method as defined in claim 4, wherein the genetic variations are selected from one or more of:
(a) a substitution and deletion mutation at 13,318,671 bp;
(b) a substitution and deletion mutation at 13,318,672 bp;
(c) a substitution and deletion mutation at 13,318,707 bp;
(d) a frame shift mutation at 13,318,709 bp;
(e) a substitution mutation at 13,318,811 bp;
(f) an insertion mutation at 13,319,691 bp;
(g) an insertion mutation at 13,319,712 bp;
(h) a substitution mutation at 13,397,560 bp;
(i) a substitution mutation at 13,409,407 bp; and
(j) a substitution mutation at 13,411,451 bp.

6. The method as defined in claim 5, wherein the genetic variations are selected from one or more of:
(a) a substitution and deletion mutation at 13,318,671 bp;
(b) a substitution and deletion mutation at 13,318,672 bp;
(c) a substitution and deletion mutation at 13,318,707 bp;
(d) a frame shift mutation at 13,318,709 bp;
(f) an insertion mutation at 13,319,691 bp; and
(g) an insertion mutation at 13,319,712 bp.

7. The method as defined in claim 5 or claim 6, wherein the genetic variations are selected from:
(b) a substitution and deletion mutation at 13,318,672 bp.

8. The method as defined in any one of claims 1 to 3, wherein the genetic variations are detected within the *CACNA1B* gene and the genetic variations are between 140,750,000 bp and 140,950,000 bp on chromosome 9.

9. The method as defined in claim 8, wherein the genetic variations are selected from one or more of:
(a) a substitution mutation at 140,772,434 bp;
(b) a substitution mutation at 140,772,440 bp;
(c) a substitution mutation at 140,772,477 bp;
(d) a substitution mutation at 140,777,306 bp; and
(e) a substitution mutation at 140,918,181 bp.

10. The method as defined in claim 9, wherein the genetic variations are selected from: (b) a substitution mutation at 140,772,440 bp.

11. A Nav1.7 sodium channel blocker for use in the treatment of trigeminal neuralgia in a patient, **characterised in that** one or more genetic variations within the *CACNA1A* gene and/or the *CACNA1B* gene have been detected in said patient.

12. The Nav1.7 sodium channel blocker for use as defined in claim 11, wherein the Nav1.7 sodium channel blocker is a Nav1.7 selective state-dependent sodium channel blocker.

13. The Nav1.7 sodium channel blocker for use as defined in claim 11 or claim 12, wherein the Nav1.7 sodium channel blocker is (5R)-5-(4-{[(2-fluorophenyl)methyl]oxy}phenyl)-L-prolinamide or a pharmaceutically acceptable salt or solvate thereof as shown in formula (I):

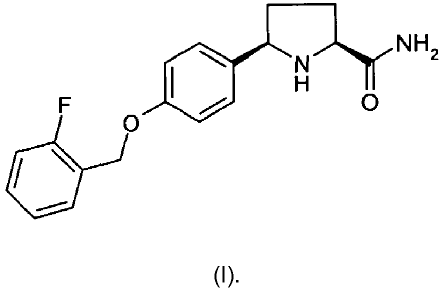

14. The Nav1.7 sodium channel blocker for use as defined in any one of claims 11 to 13, wherein the Nav1.7 sodium channel blocker is the hydrochloride salt of (5R)-5-(4-{[(2-fluorophenyl)methyl]oxy}phenyl)-L-prolinamide (CNV1014802).

## Patentansprüche

1. Verfahren zur Diagnose von Trigeminusneuralgie bei einem menschlichen Subjekt, wobei das Verfahren ein Nachweisen des Vorhandenseins einer oder mehrerer genetischer Variationen im *CACNA1A*-Gen und/oder im *CACNA1B-*Gen des Subjekts umfasst.

2. Verfahren nach Anspruch 1, wobei die genetischen Variationen Folgendes beinhalten: Mutationen (z. B. Punktmutationen), Insertionen, Substitutionen, Deletionen, Rasterschübe, Einzelnukleotidpolymorphismen (SNPs), Haplotypen, Chromosomenanomalien, Variation der Kopienanzahl (CNV), Epigenetik und DNA-Inversionen.

3. Verfahren nach Anspruch 2, wobei die genetischen Variationen aus Substitutionen, Insertionen, Deletionen und Rasterschüben ausgewählt sind.

4. Verfahren nach einem der Ansprüche 1 bis 3, wobei die genetischen Variationen im *CACNA1A*-Gen nachgewiesen werden und die genetischen Variationen zwischen Bp 13.300.000 und Bp 13.450.000 auf Chromosom 19 sind.

5. Verfahren nach Anspruch 4, wobei die genetischen Variationen ausgewählt sind aus einem oder mehreren von:
(a) einer Substitutions- und Deletionsmutation bei Bp 13.318.671;
(b) einer Substitutions- und Deletionsmutation bei Bp 13.318.672;
(c) einer Substitutions- und Deletionsmutation bei Bp 13.318.707;
(d) einem Rasterschub bei Bp 13.318.709;
(e) einer Substitutionsmutation bei Bp 13.318.811;
(f) einer Insertionsmutation bei Bp 13.319.691;
(g) einer Insertionsmutation bei Bp 13.319.712;
(h) einer Substitutionsmutation bei Bp 13.397.560;
(i) einer Substitutionsmutation bei Bp 13.409.407; und
(j) einer Substitutionsmutation bei Bp 13.411.451.

6. Verfahren nach Anspruch 5, wobei die genetischen Variationen ausgewählt sind aus einem oder mehreren von:
(a) einer Substitutions- und Deletionsmutation bei Bp 13.318.671;
(b) einer Substitutions- und Deletionsmutation bei Bp 13.318.672;
(c) einer Substitutions- und Deletionsmutation bei Bp 13.318.707;
(d) einem Rasterschub bei Bp 13.318.709;
(f) bei Insertionsmutation bei Bp 13.319.691; und
(g) bei Insertionsmutation bei Bp 13.319.712.

7. Verfahren nach Anspruch 5 oder 6, wobei die genetischen Variationen ausgewählt sind aus:
(b) einer Substitutions- und Deletionsmutation bei Bp 13.318.672.

8. Verfahren nach einem der Ansprüche 1 bis 3, wobei die genetischen Variationen im *CACNA1B-*Gen nachgewiesen werden und die genetischen Variationen zwischen Bp 140.750.000 und Bp 140.950.000 auf Chromosom 9 sind.

9. Verfahren nach Anspruch 8, wobei die genetischen Variationen ausgewählt sind aus einem oder mehreren von:
(a) einer Substitutionsmutation bei Bp 140.772.434;
(b) einer Substitutionsmutation bei Bp 140.772.440;
(c) einer Substitutionsmutation bei Bp 140.772.477;
(d) einer Substitutionsmutation bei Bp 140.777.306; und
(e) einer Substitutionsmutation bei Bp 140.918.181.

10. Verfahren nach Anspruch 9, wobei die genetischen Variationen ausgewählt sind aus:
(b) einer Substitutionsmutation bei Bp 140.772.440.

11. Nav1.7-Natriumkanalblocker zur Verwendung bei der Behandlung von Trigeminusneuralgie bei einem Patienten, **dadurch gekennzeichnet, dass** bei dem Patienten eine oder mehrere genetische Variationen im CACNA1A-Gen und/oder im *CACNA1B-*Gen nachgewiesen wurden.

12. Nav1.7-Natriumkanalblocker zur Verwendung nach Anspruch 11, der Nav1.7-Natriumkanalblocker ein Nav1.7-selektiver, stadiumabhängiger Natriumkanalblocker ist.

13. Nav1.7-Natriumkanalblocker zur Verwendung nach Anspruch 11 oder Anspruch 12, wobei der Nav1.7-Natriumkanalblocker (5R)-5-(4-{[(2-Fluorphenyl)methyl]oxy}phenyl)-L-prolinamid oder ein pharmazeutisch verträgliches Salz oder Solvat davon ist, wie in Formel (I) gezeigt:

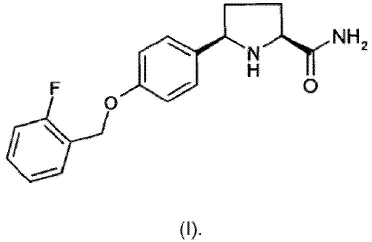

14. Nav1.7-Natriumkanalblocker zur Verwendung nach einem der Ansprüche 11 bis 13, wobei der Nav1.7-Natriumkanalblocker das Hydrochloridsalz von (5R)-5-(4-{[(2-Fluorphenyl)methyl]oxy}phenyl)-L-prolinamid (CNV1014802) ist.

## Revendications

1. Méthode de diagnostic d'une névralgie du trijumeau chez un sujet humain dans laquelle ladite méthode comprend la détection de la présence d'une ou de plusieurs variations génétiques dans le gène *CACNA1A* et/ou le gène CACNA1B dudit sujet.

2. Méthode selon la revendication 1, dans laquelle les variations génétiques incluent : des mutations (par exemple des mutations ponctuelles), des insertions, des substitutions, des délétions, des déphasages du cadre de lecture, des polymorphismes mononucléotidiques (SNP), des haplotypes, des anomalies chromosomiques, une variation du nombre de copies (CNV), l'épigénétique et des inversions d'ADN.

3. Méthode selon la revendication 2, dans laquelle les variations génétiques sont choisies parmi les substitutions, les insertions, les délétions et les déphasages du cadre de lecture.

4. Méthode selon l'une quelconque des revendications 1 à 3, dans laquelle les variations génétiques sont détectées dans le gène *CACNA1A* et les variations génétiques se situent entre la paire de bases 13 300 000 et la paire de bases 13 450 000 sur le chromosome 19.

5. Méthode selon la revendication 4, dans laquelle les variations génétiques sont choisies parmi une ou plusieurs :
(a) d'une mutation de délétion et de substitution au niveau de la paire de bases 13 318 671 ;
(b) d'une mutation de délétion et de substitution au niveau de la paire de bases 13 318 672 ;
(c) d'une mutation de délétion et de substitution au niveau de la paire de bases 13 318 707 ;
(d) d'une mutation de déphasage du cadre de lecture au niveau de la paire de bases 13 318 709 ;
(e) d'une mutation de substitution au niveau de la paire de bases 13 318 811 ;
(f) d'une mutation d'insertion au niveau de la paire de bases 13 319 691 ;
(g) d'une mutation d'insertion au niveau de la paire de bases 13 319 712 ;
(h) d'une mutation de substitution au niveau de la paire de bases 13 397 560 ;
(i) d'une mutation de substitution au niveau de la paire de bases 13 409 407 ; et
(j) d'une mutation de substitution au niveau de la paire de bases 13 411 451.

6. Méthode selon la revendication 5, dans laquelle les variations génétiques sont choisies parmi l'une ou plusieurs :
(a) d'une mutation de délétion et de substitution au niveau de la paire de bases 13 318 671 ;
(b) d'une mutation de délétion et de substitution au niveau de la paire de bases 13 318 672 ;
(c) d'une mutation de délétion et de substitution au niveau de la paire de bases 13 318 707 ;
(d) d'une mutation de déphasage du cadre de lecture au niveau de la paire de bases 13 318 709 ;
(f) d'une mutation d'insertion au niveau de la paire de bases 13 319 691 ; et
(g) d'une mutation d'insertion au niveau de la paire de bases 13 319 712.

7. Méthode selon la revendication 5 ou la revendication 6, dans laquelle les variations génétiques sont choisies parmi :
(b) une mutation de délétion et de substitution au niveau de la paire de bases 13 318 672.

8. Méthode selon l'une quelconque des revendications 1 à 3, dans laquelle les variations génétiques sont détectées dans le gène *CACNA1B* et les variations génétiques se situent entre la paire de bases 140 750 000 et la paire de bases 140 950 000 sur le chromosome 9.

9. Méthode selon la revendication 8, dans laquelle les variations génétiques sont choisies parmi une ou plusieurs :
(a) d'une mutation de substitution au niveau de la paire de bases 140 772 434 ;
(b) d'une mutation de substitution au niveau de la paire de bases 140 772 440 ;
(c) d'une mutation de substitution au niveau de la paire de bases 140 772 477 ;
(d) d'une mutation de substitution au niveau de la paire de bases 140 777 306 ; et
(e) d'une mutation de substitution au niveau de la paire de bases 140 918 181.

10. Méthode selon la revendication 9, dans laquelle les variations génétiques sont choisies parmi :
(b) une mutation de substitution au niveau de la paire de bases 140 772 440.

11. Inhibiteur du canal sodique Nav1.7 destiné à être utilisé dans le traitement de la névralgie du trijumeau chez un patient, **caractérisé en ce qu'**une ou plusieurs variations génétiques dans le gène *CACNA1A* et/ou le gène CACNA1B ont été détectées chez ledit patient.

12. Inhibiteur du canal sodique Nav1.7 destiné à être utilisé selon la revendication 11, dans lequel l'inhibiteur du canal sodique Nav1.7 est un inhibiteur de canal sodique Nav1.7 sélectif dépendant d'un état.

13. Inhibiteur du canal sodique Nav1.7 destiné à être utilisé selon la revendication 11 ou la revendication 12, dans lequel l'inhibiteur du canal sodique Nav1.7 est la (5R)-5-(4-{[(2-fluorophényl)méthyl]oxy}phényl)-L-prolinamide ou un solvate ou un sel pharmaceutiquement acceptable de cette dernière telle que présentée dans la formule (I) :

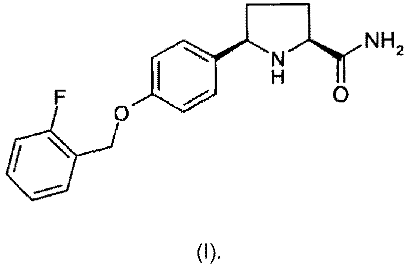

14. Inhibiteur du canal sodique Nav1.7 destiné à être utilisé selon l'une quelconque des revendications 11 à 13, dans lequel l'inhibiteur du canal sodique Nav1.7 est le sel d'hydrochlorure de (5R)-5-(4-{[(2-fluorophényl)méthyl]oxy}phényl)-L-prolinamide (CNV1014802).


### SEQUENCE LISTING

<110> Convergence Pharmaceuticals Limited
<120> DIAGNOSTIC METHOD
<130> CON-C-P1735PCT
<150> GB1409851.1
   <151> 2014-06-03
<160> 2
<170> PatentIn version 3.5
<210> 1
   <211> 21
   <212> DNA
   <213> Human
<400> 1
   cagcagcagc agcagcaggc g 21
<210> 2
   <211> 7
   <212> PRT
   <213> Human
<400> 2
   Gln Gln Gln Gln Gln Gln Ala
   1               5